# EUROPÄISCHE PATENTSCHRIFT

(11) **EP 0 667 161 B1**
(45) Veröffentlichungstag und Bekanntmachung des Hinweises auf die Patenterteilung: **17.03.1999**
(21) Anmeldenummer: 95101011.5
(22) Anmeldetag: 26.01.1995
(51) Int. Cl.: A61K 31/71, A61K 9/00

(54) **Protrahiert freisetzende Implantat-Formkörper enthaltend Clindamycin-Palmitat**
Delayed release implants made of mouldings comprising clindamycin palmitate
Implants dosés à libération retardée à partir de corps moules contenant de la clindamycine palmitate

(30) Priorität: 09.02.1994 DE 4404018
(43) Veröffentlichungstag der Anmeldung: 16.08.1995
(73) Patentinhaber: MERCK PATENT GmbH, 64293 Darmstadt (DE)
(72) Erfinder: Cimbollek, Monika, Dr., D-68167 Mannheim (DE); Nies, Berthold, Dr., D-64407 Fränkisch-Crumbach (DE)

(56) Entgegenhaltungen:
- WO-A-92/15286
- WO-A-92/18101
- US-A- 4 539 234
- US-A- 5 202 128
- J. ANTIMICROB. CHEMOTHERAP., Bd. 7, no.SuppA, 1981 Seiten 3-9, D.A. LEIGH 'Antibacterial activity and pharmacokinetics of clindamycin'

## Beschreibung

Die Erfindung betrifft die Verwendung von Clindamycin-Palmitat zur Herstellung von Implantat-Formkörpern mit protrahierter Freisetzung des antibiotischen Wirkstoffes Clindamycin.

Clindamycin ist ein Antibiotikum von allgemein anerkanntem Wert. Es entfaltet besondere Effektivität gegen gram-positive Organismen wie Staphylokokken und Streptokokken sowie gegen gram-negative Anaerobier. Daher wird dieses Antibiotikum zur Behandlung eines weiten Krankheitsspektrums verwendet, wie z. B. bei der Bekämpfung von Infektionen des Verdauungstraktes, der Haut und der Weichgewebe sowie bei Osteomyelitis und bei gynäkologischen Infektionen. Weiterhin ist Clindamycin erfolgreich bei Prophylaxe und Therapie bakterieller Endokarditis eingesetzt worden.

Bei den genannten Indikationen kommt dieses Antibiotikum in Form physiologisch akzeptabler Salze wie beispielsweise Clindamycin · HCl, des Clindamycin-Phosphatesters oder auch in Form der freien Clindamycin-Base zur Anwendung.

Clindamycin ist auch in Form des Palmitat-Esters bekannt. Clindamycin-Palmitat selbst ist mikrobiologisch inaktiv. Allerdings wird es durch Enzyme des Dünndarmes leicht hydrolysiert und aus dieser Form die aktive Clindamycin-Base freigesetzt. Die Plasmahalbwertzeit des aktiven Wirkstoffes beträgt hierbei ca. 2 Stunden. Clindamycin-Palmitat hat aufgrund dieser Eigenschaft in Form des leicht in wäßrigem Medium löslichen HCl-Salzes Anwendung als rasch und kurzzeitig wirkendes Antibiotikum in Therapieformen mit oraler Verabreichung des Wirkstoffes gefunden. Der freie Clindamycin-Palmitat-Ester (also nicht in Form des HCl-Salzes) ist in wäßrigen Medien praktisch unlöslich und blieb daher bislang pharmakologisch unbedeutsam.

In D.A. Leigh et al., J. Antimicrobial Chemotherapy (1981), 7, Suppl. A3-9, wird nach oraler Gabe für Clindamycin-Palmitat die gleiche Kinetik wie für Clindamycin-HCl beschrieben.

In US4, 539, 234, W092/15286 und W092/18101 ist die Verwendung relevanter Wirkstoffe, u.a. Clindamycin-Palmitat Hydrochlorid als Depotformen unter Zuhilfenahme von chemischer Kopplung oder von Coatings beschrieben.

Überraschend wurde nun gefunden, daß die Hydrolyse von Clindamycin-Palmitat in das aktive Clindamycin nicht nur im Gastrointestinaltrakt, sondern auch an anderen Stellen des Körpers unter dem Einfluß von Körperflüssigkeiten wie Serum und Blut erfolgen kann. Mittels in-vitro-Experimenten konnte festgestellt werden, daß in Serum oder Vollblut oder unter dem Einfluß von Makrophagen aus Clindamycin-Palmitat die aktive Clindamycin-Base langsam, über einen Zeitraum von Tagen bis Wochen hinweg, mit in etwa konstanter Rate freigesetzt wird. Aufgrund dieser Befunde ergibt sich die grundsätzliche Eignung von Clindamycin-Palmitat an sich als lokal, beispielsweise an Operationswunden, zur Prophylaxe und Therapie von Infektionen einzusetzendes Depot-Antibiotikum mit protrahierter Freisetzung des Wirkstoffes Clindamycin.

Im besonderen Maße eignet sich Clindamycin-Palmitat für die Herstellung von Implantat-Formkörpern mit protrahierter Freisetzung des antibiotischen Wirkstoffes Clindamycin.

Gegenstand der Erfindung ist somit die Verwendung von Clindamycin-Palmitat zur Herstellung von Implantat-Formkörpern mit protrahierter Freisetzung des antibiotischen Wirkstoffes Clindamycin.

Gegenstand der Erfindung sind weiterhin Implantat-Formkörper mit protrahierter Freisetzung des antibiotischen Wirkstoffes Clindamycin, wobei diese Clindamycin-Palmitat enthalten.

Die erfindungsgemäße Eignung von Clindamycin-Palmitat als Depot-Antibiotikum für die Herstellung von Implantat-Formkörpern mit protrahierter Wirkstofffreisetzung ergibt sich einerseits aus dessen geringer Löslichkeit in wäßrigen bzw. physiologischen Medien und andererseits aus der bislang nicht entdeckten Zugänglichkeit durch metabolisierende Vorgänge in Körperflüssigkeiten, durch die die aktive Clindamycin-Base freigesetzt wird und ihre antibiotische Wirkung entfalten kann. So ergibt sich beispielsweise aus in-vitro-Untersuchungen in Rattenblut nach einer Inkubationszeit von 4 Stunden eine Hydrolyse von Clindamycin-Palmitat zu 70 % und nach 24 Stunden zu 100 %. In Humanserum und in Pferdeserum sind nach diesem Zeitraum 35 % des Esters in die aktive Clindamycin-Base gespaltet. In-vivo-Experimente an Ratten, denen mit Clindamycin-Palmitat imprägnierte PTFE-Vliesstücke in den Oberschenkelmuskel implantiert wurden, zeigten über einen Zeitraum von 14 Tagen hinweg eine konstante bis leicht ansteigende Ausscheidung von Clindamycin-Base im Urin der Tiere, während Clindamycin-Palmitat nicht gefunden werden konnte. Außerdem konnte Clindamycin-Base, in dem das Implantat umgebenden Muskelgewebe, nachgewiesen werden.

Erfindungsgemäß kann demnach Clindamycin-Palmitat vorteilhaft in Implantat-Formkörpern mit protrahierter Freisetzung des Wirkstoffes Clindamycin eingesetzt werden. Vornehmlich dienen diese Implantat-Formkörper dem Knochenersatz bei der Behandlung bzw. Rekonstitution von unfall- oder krankheitsbedingten Knochendefekten. Weiterhin können entsprechende Implantat-Formkörper auch für den Ersatz anderer Organteile dienen, wie etwa als künstliche Herzklappen, Herzklappen-Nahtringe, künstliche Blutgefäße oder sonstiges chirurgisches Nahtmaterial. Implantat-Formkörper können aber auch nur als Depot-Implantate zur lokalen Wirkstofffreisetzung bei der Bekämpfung oder Prophylaxe von Infektionen dienen.

Zweckmäßgerweise enthalten derartige Implantat-Formkörper 5 bis 250 mg/ cm³ an Clindamycin-Palmitat. In diesem Konzentrationsbereich ist für die gängigen Indikationen eine über einen ausreichenden Zeitraum anhaltende Freisetzung wirksamer Mengen an Clindamycin gewährleistet.

Vorteilhaft ist die Kombination von Clindamycin-Palmitat mit einem oder mehreren weiteren pharmazeutischen Wirkstoffen, insbesondere solchen mit ebenfalls antibiotischer Wirkung. Hierbei können das Wirkungsspektrum ausgeweitet und/oder zeitlich und mengenmäßig unterschiedliche Freisetzungsraten der verschiedenen Antibiotika kombiniert werden.

Beispiele für derartige mit Clindamycin-Palmitat kombinierbare weitere Antibiotika sind Gentamicin-Sulfat und insbesondere das Salz von Gentamicin mit 3-p-Methoxybenzyliden-6-hydroxy-4'-methoxyflavanon-6-phosphat. Letzteres Gentamicin-Salz ist schwerlöslich und stellt daher eine Depotform von Gentamicin mit protrahierter Freisetzung dar.

Der erfindungsgemäße Einsatz von Clindamycin-Palmitat zur Herstellung von Implantat-Formkörpern mit protrahierter Freisetzung kann zweckmäßigerweise in der Art erfolgen, daß man ein geeignetes Trägermaterial, das in Gestalt eines Implantat-Formkörpers vorliegt oder zu einem solchen weiter verarbeitet werden kann, mit Clindamycin-Palmitat imprägniert, so daß dessen freie äußere und gegebenenfalls innere Oberfläche und/oder das Matrixmaterial selbst möglichst gleichmäßig mit dem Wirkstoff beladen wird. Zweckmäßigerweise erfolgt diese Imprägnierung mit einer flüssigen, den Implantat-Formkörper gut benetzenden bzw. durchdringenden Präparation von Clindamycin-Palmitat. Eine solche flüssige Präparation kann eine Suspension oder vorzugsweise eine Lösung des Wirkstoffes sein.

Es hat sich herausgestellt, daß ein Gemisch aus 7 bis 9 Volumenteilen Tetrahydrofuran und 3 bis 1 Volumenteilen Wasser ein besonders gutes Lösungsmittel für Clindamycin-Palmitat darstellt. In derartigen Tetrahydrofuran-Wasser-Gemischen läßt sich Clindamycin bis zu etwa 33 Gew.-% lösen. Derartige Lösungsmittelgemische sind auch besonders vorteilhaft, wenn Clindamycin-Palmitat in Kombination mit der vorgenannten Depotform von Gentamicin eingesetzt werden soll. Gemäß der Deutschen Patentanmeldung P 43 14 871 sind derartige Gemische vorzügliche Lösungsmittel für dieses ansonsten nur schwerlösliche Gentamicin-Salz.

Als Trägermaterialien für Clindamycin-Palmitat und eventuelle weitere pharmazeutische Wirkstoffe kommen in erster Linie poröse Materialien in Frage, die die Wirkstofflösung gut aufsaugen können. Bevorzugt sind bioaktive und insbesondere bioresorbierbare Materialien, die vorzugsweise in Form von Implantat-Formkörpern vorliegen oder zu solchen verarbeitet werden können. Typische poröse Biomaterialien für Implantat-Formkörper sind Calciumphosphate wie insbesondere Calciumphosphatkeramiken. In der Regel bestehen diese aus Hydroxylapatit synthetischen oder natürlichen Ursprungs. Bevorzugt sind Knochenkeramiken, die aus natürlichen Knochen durch Entfernung des organischen Anteils und Sintern der Mineralphase zur Keramik gewonnen werden können. Besonders geeignet für die Imprägnierung mit Wirkstoff ist Spongiosakeramik aufgrund der naturbedingten hohen Porosität. Weitere Calciumphosphatmaterialien sind Tricalciumphosphat und Tetracalciumphosphat, die sinngemäß eingesetzt werden.

Poröse Implantat-Formkörper aus bioinerten Polymermaterialien wie beispielsweise Polytetrafluorethylen ("Teflon") oder aus Biopolymeren wie Kollagen, Gelatine, Chitin, Chitosan, Polylactiden oder Polyglykoliden können ebenfalls in erfindungsgemäßer Weise mit Clindamycin-Palmitat imprägniert werden. Vorzugsweise liegen diese Materialien in Form von saugfähigen Gebilden mit schwammartiger, vliesartiger oder Gewebestruktur oder als Gele bzw. wachsartige Massen vor. Poröse Verbundmaterialien aus Biokeramiken und Biopolymeren eignen sich ebenfalls als Wirkstoffträger.

Die Beladung der porösen Wirkstoffträger mit Clindamycin-Palmitat erfolgt zweckmäßigerweise in der Art, daß man den Träger mit der Wirkstofflösung durch vollständiges Eintauchen oder Betropfen behandelt bis das aufnahmefähige Volumen vollständig mit der Lösung gefüllt ist. Danach wird der Träger getrocknet, vorzugsweise in einem warmen Luftstrom. Die Trocknung erfolgt relativ rasch, da das Tetrahydrofuran, das den Hauptanteil des Lösungsmittelgemisches ausmacht, schnell verdunstet. Hierdurch erhalten die inneren und äußeren Oberflächen der Formkörper eine weitestgehend gleichmäßige Beschichtung mit dem Wirkstoff. Nach einer gegebenenfalls erforderlichen Sterilisation und Sterilverpackung sind die mit dem Wirkstoff beladenen Implantat-Formkörper gebrauchsfertig.

Durch eine sinngemäße Behandlung mit der Clindamycin-Palmitat-Lösung können auch unporöse oder nur oberflächenrauhe Implantate mit einer Wirkstoffschicht überzogen werden. Eine derartige antibiotische Beschichtung mit protrahierter Wirkstofffreisetzung kann vorteilhaft für viele Arten von Endoprothesen aus Metall, Keramik oder Kunststoff sein. Neben der hohen Wirkstoffkonzentration der Lösung zeigt diese hierbei noch den Vorteil, daß sie diese Materialien gut benetzt und gleichmäßig überzieht. Vorteilhaft ist beispielsweise die antibiotische Beschichtung des Femurschaftes von Hüftendoprothesen sowie die antibiotische Imprägnierung von Herzklappen-Nahtringen.

In-vitro und in-in-vivo-Experimente mit Implantat-Formkörpern die Clindamycin-Palmitat enthalten, zeigen eine langanhaltende, gleichmäßige Abgabe der antibiotisch wirksamen Clindamycin-Base, während eine Abgabe des unwirksamen Clindamycin-Palmitat nicht oder nur in geringem Umfang zu verzeichnen ist.

### Beispiel 1

Aus einer Lösung von 1 g Clindamycin-Palmitat-HCl (Firma Upjohn, USA) in 25 ml Wasser wird durch Zugabe von 10 ml einer 0,1 % normalen NaOH-Lösung der Clindamycin-Palmitat-Ester ausgefällt, abfiltriert, mit Wasser gewaschen und getrocknet.

### Beispiel 2

0,1 g des Clindamycin-Palmitat-Esters nach Beispiel 1 werden in 100 ml einer 1,5%igen flüssigen Agar-Lösung suspendiert. Nach Verfestigung werden aus der Masse Formkörper von ca. 0,725 cm³ geschnitten.

Diese Formkörper werden in frischem, aktivem Humanserum eluiert. Die Clindamycin-Aktivitäten in den Eluaten werden mikrobiologisch bestimmt. Die Ergebnisse zeigen über mehr als 20 Tage eine Freisetzung nullter Ordnung bei einer Konzentration von ca. 10 µg/ml.

### Beispiel 3

Clindamycin-Palmitat-Ester nach Beispiel 1 wird in einem Gemisch aus 9 Volumenteilen THF und 1 Volumenteil H₂O unter Rühren bei 25 °C zu einer Lösung enthaltend 0,33 g/ml aufgelöst.

### Beispiel 4

Blöcke von aus natürlicher Spongiosa hergestellten porösen Hydroxylapatitkeramiken der Abmessungen 12,5 x 12,5 x 10 mm werden durch Eintauchen mit der Lösung nach Beispiel 3 getränkt. Die mit Lösung vollgesogenen Keramikblöcke werden entnommen und innerhalb von 2 Stunden im warmen Luftstrom getrocknet. Die gebrauchsfertigen Blöcke enthalten ca. 120 mg an Clindamycin-Palmitat

### Beispiel 5

Kreisförmige Teflonvliese mit Durchmesser 0,5 cm und Dicke 0,2 cm werden in die Lösung nach Beispiel 3 eingetaucht bis sie sich vollständig vollgesogen haben. Danach werden sie entnommen und ca. 30 Minuten im warmen Luftstrom getrocknet. Die gebrauchsfertigen Vliese enthalten 25 mg Clindamycin-Palmitat.

### Beispiel 6

Teflonvliese nach Beispiel 5 werden in die Oberschenkelmuskel von 6 Ratten impantiert. Die Tiere werden in Metabolitenkäfigen gehalten. Die Freisetzung von Antibiotikum wird täglich innerhalb eines 2-Wochen-Zeitraumes im gesammelten Urin bestimmt.

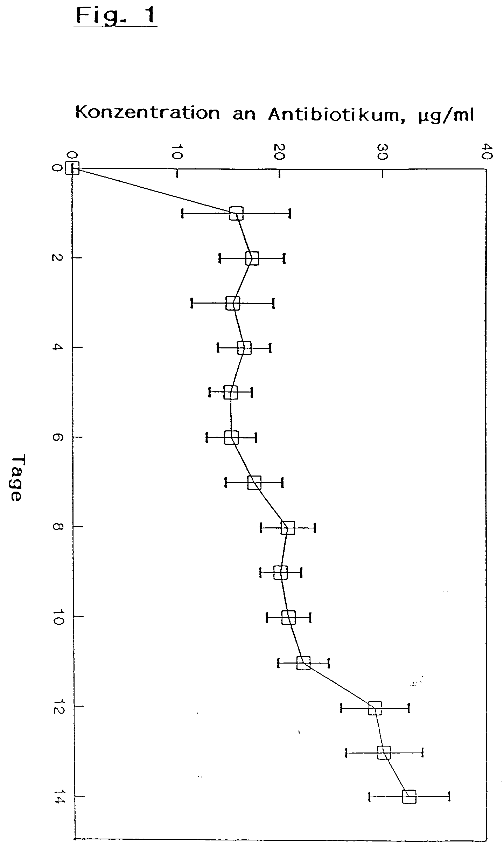

Figur 1 zeigt das Ergebnis.

Über den Versuchszeitraum wurde kein Clindamycin-Palmitat im Urin nachgewiesen, jedoch ein von 16 bis 32 µg/ml leicht ansteigender Gehalt an antibiotisch wirksamen Clindamycin.

### Beispiel 7

Segmente von Herzklappen-Nahtringen (Firma Sorin, Italien) werden zunächst mit einer Lösung aus 9 Volumenteilen THF und 1 Volumenteil H₂O enthaltend 0,33 g/ml Clindamycin-Palmitat und 0,1 g/ml des Salzes von Gentamicin mit 3-p-Methoxybenzyliden-6-hydroxy-4'-methoxyflavanon-6-phosphat getränkt und getrocknet. Die Segmente werden dann in einer wäßrigen Lösung enthaltend 0,03 g/ml Gentamicin-Sulfat getaucht und erneut getrocknet. Man erhält auf diese Weise Nahtringsegmente, die 20 mg Clindamycin-Palmitat, 14 mg des schwerlöslichen Gentamicin-Salzes und 4 mg Gentamicin-Sulfat enthalten.

Diese Segmente werden in die Oberschenkelmuskel von 5 Ratten implantiert. Die Tiere werden in Metabolitenkäfigen gehalten. Die Freisetzung von Antibiotikum wird täglich innerhalb eines 2-Wochen-Zeitraumes im gesammelten Urin bestimmt.

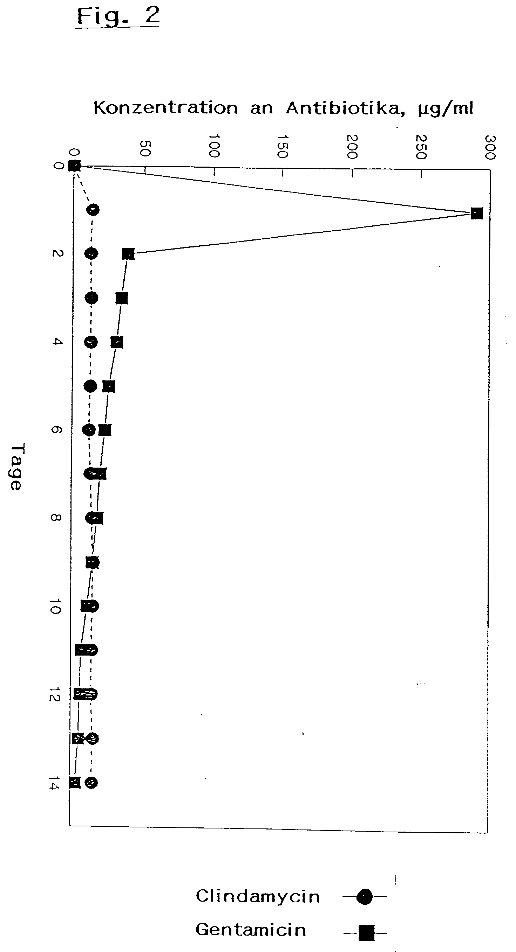

Figur 2 zeigt das Ergebnis.

Über den Versuchszeitraum wurde kein Clindamycin-Palmitat im Urin nachgewiesen, jedoch ein konstanter Gehalt von ca. 14 µg/ml an Clindamycin. Die Freisetzungskurve für Gentamicin zeigt innerhalb der ersten 24 Stunden einen initialen Spitzenwert von 290 µg/ml, der von einer protrahierten von 38 auf 3,5 µg/ml abfallenden Freisetzung gefolgt ist.

## Patentansprüche

1. Verwendung von Clindamycin-Palmitat zur Herstellung von Implantat-Formkörpern mit protrahierter Freisetzung des antibiotischen Wirkstoffes Clindamycin.

2. Implantat-Formkörper mit protrahierter Freisetzung des antibiotischen Wirkstoffes Clindamycin, dadurch gekennzeichnet, daß er Clindamycin-Palmitat enthält.

3. Implantat-Formkörper nach Anspruch 2, dadurch gekennzeichnet, daß dieser 5 bis 250 mg/cm³ an Clindamycin-Palmitat enthält.

4. Implantat-Formkörper nach Anspruch 3, dadurch gekennzeichnet, daß dieser Clindamycin-Palmitat in Kombination mit einem oder mehreren weiteren pharmazeutischen Wirkstoffen, insbesondere mit antibiotischer Wirkung, enthält.

5. Implantat-Formkörper nach Anspruch 4, dadurch gekennzeichnet, daß dieser Clindamycin-Palmitat in Kombination mit Gentamycin-Sulfat und/oder mit dem Salz von Gentamycin mit 3-p-Methoxy-benzyliden-6-hydroxy-4'-methoxyflavanon-6-phosphat enthält.

6. Implantat-Formkörper nach den Ansprüchen 2-5, dadurch gekennzeichnet, daß dieser aus einem Wirkstoffträger besteht, der mit Clindamycin-Palmitat imprägniert ist.

7. Implantat-Formkörper nach Anspruch 6, dadurch gekennzeichnet, daß der Wirkstoffträger aus einem bioinerten oder aus einem bioresorbierbaren Polymermaterial mit schwammartiger, vließartiger oder Gewebestruktur besteht.

8. Implantat-Formkörper nach Anspruch 6, dadurch gekennzeichnet, daß der Wirkstoffträger aus einem porösen keramischen Material, insbesondere auf Basis von Calciumphosphat, besteht.

9. Verfahren zur Herstellung von Implantat-Formkörpern mit protrahierter Freisetzung des antibiotischen Wirkstoffes Clindamycin, dadurch gekennzeichnet, daß man Clindamycin-Palmitat und gegebenenfalls weitere pharmazeutische Wirkstoffe in einem Gemisch aus 7-9 Volumenteilen Tetrahydrofuran und 3-1 Volumenteilen Wasser löst, mit dieser Lösung einen Wirkstoffträger behandelt, und dann das Lösungsmittel durch Trocknung entfernt.

## Claims

1. Use of clindamycin palmitate for the production of shaped implants having a protracted release of the antibiotic active compound clindamycin.

2. Shaped implant having a protracted release of the antibiotic active compound clindamycin, characterized in that it contains clindamycin palmitate.

3. Shaped implant according to Claim 2, characterized in that this contains 5 to 250 mg/cm³ of clindamycin palmitate.

4. Shaped implant according to Claim 3, characterized in that this contains clindamycin palmitate in combination with one or more further pharmaceutical active compounds, in particular having antibiotic action.

5. Shaped implant according to Claim 4, characterized in that this contains clindamycin palmitate in combination with gentamycin sulphate and/or with the salt of gentamycin with 3-p-methoxybenzylidene-6-hydroxy-4'-methoxyflavanone-6-phosphate.

6. Shaped implant according to Claims 2-5, characterized in that this consists of an active compound carrier which is impregnated with clindamycin palmitate.

7. Shaped implant according to Claim 6, characterized in that the active compound carrier consists of a bioinert or of a bioabsorbable polymer material having a spongy, nonwoven or woven fabric structure.

8. Shaped implant according to Claim 6, characterized in that the active compound carrier consists of a porous ceramic material, in particular based on calcium phosphate.

9. Process for the production of shaped implants having a protracted release of the antibiotic active compound clindamycin, characterized in that clindamycin palmitate and if desired further pharmaceutical active compounds are dissolved in a mixture of 7-9 parts by volume of tetrahydrofuran and 3-1 parts by volume of water, an active compound carrier is treated with this solution, and the solvent is then removed by drying.

## Revendications

1. Utilisation de palmitate de clindamycine pour préparer des objets façonnés pour implantation, présentant une, libération retardée du principe actif antibiotique clindamycine.

2. Objet façonne pour implantation, à libération retardée du principe actif antibiotique clindamycine, caractérisé en ce qu'il contient du palmitate de clindamycine.

3. Objet façonné pour implantation selon la revendication 2, caractérisé en ce qu'il contient de 5 à 250 mg/cm³ de palmitate de clindamycine.

4. Objet façonné pour implantation selon la revendication 3, caractérisé en ce qu'il contient du palmitate de clindamycine en combinaison avec un ou plusieurs autres principes actifs pharmaceutiques, en particulier ayant un effet antibiotique.

5. Objet façonné pour implantation selon la revendication 4, caractérisé en ce qu'il contient du palmitate de clindamycine en combinaison avec du sulfate de gentamicine et/ou avec le sel de la gentamicine obtenu avec le 3-p-méthoxy-benzylidène-6-hydroxy-4'-méthoxyflavanone-6-phosphate.

6. Objet façonné pour implantation selon les revendications 2 à 5, caractérise en ce qu'il est constitue d'un support pour principe actif qui est imprégné de palmitate de clindamycine.

7. Objet façonné pour implantation selon la revendication 6, caractérisé en ce que le support pour principe actif est constitué d'un matériau polymère bio-inerte ou biorésorbable, ayant une structure spongieuse, ou la structure d'un tissu ou d'un non-tissé.

8. Objet façonné pour implantation selon la revendication 6, caractérisé en ce que le support pour principe actif est constitué d'un matériau céramique poreux, en particulier à base de phosphate de calcium.

9. Procédé de préparation d'objets façonnés pour implantation à libération retardée pour principe actif antibiotique clindamycine, caractérisé en ce qu'on dissout du palmitate de clindamycine et éventuellement d'autres principes actifs pharmaceutiques dans un mélange de 7 à 9 parties en volume de tétrahydrofuranne et de 3 à 1 parties en volume d'eau, on traite avec cette solution un support de principe actif, puis on élimine le solvant par séchage.
